# EUROPEAN PATENT APPLICATION

(11) **EP 4 559 579 A1**
(43) Date of publication of application: **28.05.2025**
(21) Application number: 23842850.2
(22) Date of filing: 07.07.2023
(51) Int. Cl.: B03B 5/00, C12M 1/00, G01N 37/00

(54) **SEPARATION DEVICE**

(30) Priority: 21.07.2022 JP 2022116505
(71) Applicant: SCREEN Holdings Co., Ltd., Kyoto-shi, Kyoto 602-8585 (JP)
(72) Inventor: URAKAWA, Satoshi, Kyoto-shi, Kyoto 602-8585 (JP)
(74) Representative: Goddar, Heinz J.
(86) International application number: PCT/JP2023/025288
(87) International publication number: WO 2024/018925

(57) **Abstract**

A separation device (100) includes a main channel (11), a plurality of auxiliary channels (13), a control liquid channel (21), a downstream side joining portion (23), and a common channel (25). A sample liquid (L1) containing particles flows through the main channel (11). The plurality of auxiliary channels (13) branch from the main channel (11). The control liquid channel (21) joins to a portion of the main channel (11) which is located on a downstream side of the auxiliary channels (13), and a control liquid (L3) flows through the control liquid channel (21). At the downstream side joining portion (23), the main channel (11) and the control liquid channel (21) join. The common channel (25) is connected to the downstream side joining portion (23). At least the particles larger than a predetermined particle size and the control liquid (L3) flow through the common channel (25).

## Description

### Technical Field

The present invention relates to a separation device.

### Background Art

Conventionally, a separation device that separates specific cells from blood has been known (See, for example, Patent Literature 1.). Patent Literature 1 describes a channel chip including a main channel and a plurality of branch channels branching from the main channel. The channel chip further includes two inlet channels coupled to one end of the main channel and an outlet channel coupled to the other end of the main channel.

Blood serving as a sample liquid is made to flow to one inlet channel. A buffer liquid flows through the other inlet channel. As a result, the blood and the buffer liquid flow into one end of the main channel. Then, the cancer cells present in the blood reach the other end of the main channel and flow through the outlet channel. Meanwhile, blood components other than the cancer cells flow to the plurality of branch channels. In such a way, the cancer cells are separated from the blood. It is noted that part of the buffer liquid flows into the plurality of branch channels, and the rest of the buffer liquid reaches the other end of the main channel and flows through the outlet channel.

### Citation List

### Patent Literature

Patent Literature 1: Japanese Patent Application Publication No. 2020-99256

### Summary of Invention

### Technical Problem

Incidentally, in the channel chip of Patent Literature 1, for example, when the cross-sectional dimension of each channel is formed larger or smaller than the set value even by 1 µm or less, the channel resistance of each part becomes smaller or larger than the set value by several 10% or more. In this case, cancer cells may flow into the branch channels, or particles smaller than cancer cells or a liquid component of blood may flow into the outlet channel. That is, there is a problem that cancer cells cannot be separated from blood in some cases.

The present invention has been made in view of the above problems, and an object of the present invention is to provide a separation device that can separate particles having a predetermined particle size even when the dimension of a channel deviates from a set value.

### Solution to Problem

A separation device according to one aspect of the present invention includes a main channel, a plurality of auxiliary channels, a control liquid channel, a first joining portion, and a common channel. A sample liquid containing particles flows through the main channel. The plurality of auxiliary channels branch from the main channel. The control liquid channel is joined to a portion of the main channel which is located on a downstream side of the auxiliary channels. A control liquid flows through the control liquid channel. At the first joining portion, the main channel and the control liquid channel join. The common channel is connected to the first joining portion. At least the particles larger than a predetermined particle size and the control liquid flow through the common channel.

In a mode of the present invention, a control liquid adjusting portion capable of adjusting an amount of the control liquid flowing through the control liquid channel may further be included.

In a mode of the present invention, a sample liquid sending portion that causes the sample liquid to flow into the main channel may further be included. The control liquid adjusting portion and the sample liquid sending portion may be driven independently of each other.

In a mode of the present invention, a sample liquid supply channel, a transport liquid supply channel, a second joining portion, and a transport liquid sending portion may further be included. The sample liquid may flow through the sample liquid supply channel. A transport liquid that transports the particles to the common channel may flow through the transport liquid supply channel. At the second joining portion, the sample liquid supply channel and the transport liquid supply channel may join. The transport liquid sending portion may cause the transport liquid to flow into the transport liquid supply channel. The transport liquid sending portion and the control liquid adjusting portion may be driven independently of each other.

In a mode of the present invention, the control liquid and the transport liquid may be the same in components.

In a mode of the present invention, a width of the common channel may be larger than a sum of a width of the main channel and a width of the control liquid channel. Advantageous Effects of Invention

The present invention can provide a separation device that can separate particles having a predetermined particle size even when the dimension of a channel deviates from a set value.

### Brief Description of Drawings

[FIG. 1] is a plan view schematically illustrating a structure of a separation device according to a preferred embodiment of the present invention.
[FIG. 2] is a plan view schematically illustrating a structure around an upstream side joining portion of the separation device.
[FIG. 3] is a schematic view for explaining an operating principle of an HDF.
[FIG. 4] is a conceptual diagram for explaining channel resistance acting on a sample liquid and a transport liquid in the main channel, the auxiliary channels, and the common channel.
[FIG. 5] is a schematic view for explaining a relationship between a supply amount of control liquid and an amount of transport liquid flowing through the common channel.
[FIG. 6] is a schematic view for explaining a relationship between a supply amount of control liquid and an amount of transport liquid flowing through the common channel.
[FIG. 7] is a plan view schematically showing a structure of a separation device according to a modified example of the present invention.

### Description of Embodiments

Hereinafter, a preferred embodiment of the present invention will be described with reference to the drawings. It is noted that in the drawings, the same or corresponding portions will be given the same reference signs and will not be repeatedly described.

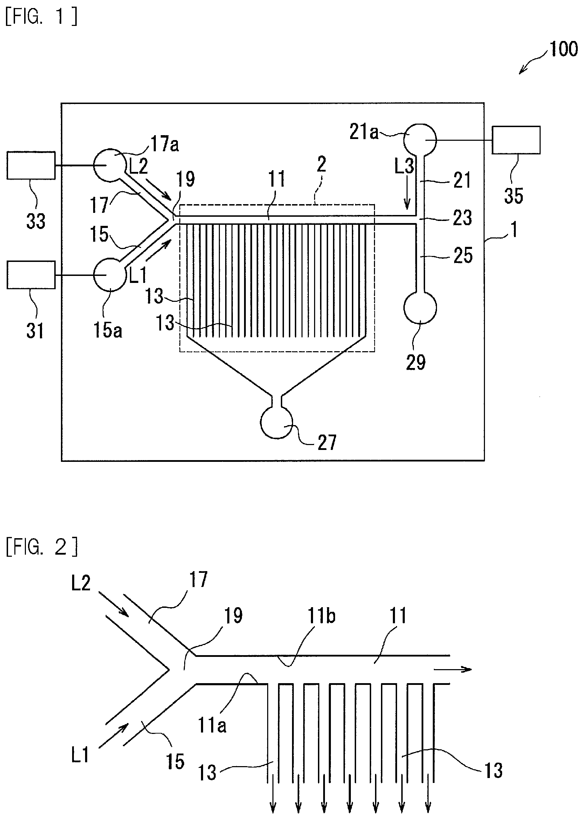

With reference to FIGS. 1 to 6, a separation device 100 according to a preferred embodiment of the present invention will be described. FIG. 1 is a plan view schematically illustrating a structure of the separation device 100 according to a preferred embodiment of the present invention.

As illustrated in FIG. 1, the separation device 100 of the present preferred embodiment includes a hydrodynamic filtration (HDF) chip 1. The HDF chip 1 has a function as a hydrodynamic filter. For example, the HDF chip 1 is a microchannel chip for the purpose of separating and concentrating fine particles. Also, for example, the HDF chip 1 is constituted of a transparent substrate and the like.

The HDF chip 1 includes a main channel 11, a plurality of auxiliary channels 13, a sample liquid supply channel 15, a transport liquid supply channel 17, an upstream side joining portion 19, a control liquid channel 21, a downstream side joining portion 23, and a common channel 25. The main channel 11, the plurality of auxiliary channels 13, the sample liquid supply channel 15, the transport liquid supply channel 17, the upstream side joining portion 19, the control liquid channel 21, the downstream side joining portion 23, and the common channel 25 are formed by resin layers and the like disposed on a transparent substrate, for example. It is noted that the upstream side joining portion 19 is a "second joining portion" of the present invention. Also, the downstream side joining portion 23 is an example of a "first joining portion" of the present invention.

The main channel 11 extends in a straight-line form toward, for example, a predetermined direction. A sample liquid L1 flows through the main channel 11. In the present preferred embodiment, the sample liquid L1 and a transport liquid L2 flow through the main channel 11. The sample liquid L1 is a liquid containing fine particles. The sample liquid L1 is not particularly limited but is blood, for example. The transport liquid L2 is a liquid not containing fine particles, for example. The transport liquid L2 is not particularly limited but is a liquid culture media, for example.

The plurality of auxiliary channels 13 branch from the main channel 11. Specifically, the plurality of auxiliary channels 13 are disposed at substantially equal pitches along the direction in which the main channel 11 extends, for example. The plurality of auxiliary channels 13 have the same length, for example. The plurality of auxiliary channels 13 extend in a direction intersecting the main channel 11. In the present preferred embodiment, the plurality of auxiliary channels 13 extend in a direction orthogonal to the main channel 11.

Also, the plurality of auxiliary channels 13 are formed so that channel resistance is gradually reduced from the upstream side of the main channel 11 toward the downstream side. Specifically, in the present preferred embodiment, a portion on the proximal end side (a side close to the main channel 11) of each of the auxiliary channels 13 is formed to have a first width. Meanwhile, a portion of each auxiliary channel 13 which is located on the terminal side (the side far from the main channel 11) is formed to have a second width larger than the first width. Then, the position where the width of the auxiliary channel 13 changes from the first width to the second width is arranged closer to the proximal end side as the auxiliary channel is closer to the downstream side joining portion 23. The first width is not particularly limited but is, for example, about 10 µm to several 10 µm. The second width is not particularly limited but is about several 10 µm to 100 µm, for example. It is noted that all of the auxiliary channels 13 may be formed to have the same constant width. In this case, for example, the auxiliary channel 13 closer to the downstream side joining portion 23 may be formed shorter.

An HDF 2 is constituted of the main channel 11 and the plurality of auxiliary channels 13. Specifically, part of the liquid flowing through the main channel 11 flows into the plurality of auxiliary channels 13. Also, part of the particles contained in the liquid flowing through the main channel 11 flows into the plurality of auxiliary channels 13. Whether or not the particles flow into the plurality of auxiliary channels 13 depends on the particle size, for example. The particles having the smaller particle sizes more easily flow into the auxiliary channel 13, and the particles having the larger particle sizes less easily flow into the auxiliary channel 13. For example, in a case where blood is used as the sample liquid L1, it is possible to make blood plasma (also called as plasma) serving as a liquid component of blood and red blood cells and blood platelets flow through the auxiliary channel 13, and to inhibit cancer cells and white blood cells contained in blood from flowing through the auxiliary channel 13. That is, it is possible to separate the particles having the large particle sizes, such as cancer cells and the white blood cells, from blood. It is noted that an operating principle of the HDF 2 will be described later.

FIG. 2 is a plan view schematically illustrating a structure around the upstream side joining portion 19 of the separation device 100. As shown in FIG. 2, the sample liquid L1 flows through the sample liquid supply channel 15. The sample liquid supply channel 15 is a channel for supplying the sample liquid L1 to the main channel 11. It is noted that although the sample liquid supply channel 15 is inclined with respect to the main channel 11 in the present preferred embodiment, the present invention is not limited to this. For example, the sample liquid supply channel 15 may be parallel to the main channel 11 or may be perpendicular to the main channel 11.

The transport liquid L2 flows through the transport liquid supply channel 17. The transport liquid supply channel 17 is a channel for supplying the transport liquid L2 to the main channel 11. The transport liquid L2 is, for example, a liquid for transporting particles having a particle size larger than a predetermined particle size to the common channel 25. In other words, the transport liquid L2 is a liquid for transporting the particles not flowing into the auxiliary channels 13 to the common channel 25. It is noted that although the transport liquid supply channel 17 is inclined with respect to the main channel 11 in the present preferred embodiment, the present invention is not limited to this. For example, the transport liquid supply channel 17 may be parallel to the main channel 11 or may be perpendicular to the main channel 11.

Also, the sample liquid supply channel 15 is disposed so that the sample liquid L1 flows through a portion of the main channel 11 on the auxiliary channel 13 side. Meanwhile, the transport liquid supply channel 17 is disposed so that the transport liquid L2 flows through a portion of the main channel 11 on the opposite side to the auxiliary channels 13. In the present preferred embodiment, the sample liquid supply channel 15 is connected to an inner wall 11a of the inner wall of the main channel 11 which is located on the auxiliary channel 13 side. On the other hand, the transport liquid supply channel 17 is connected to an inner wall 11b of the inner wall of the main channel 11 which is located on the opposite side to the auxiliary channels 13.

In the upstream side joining portion 19, the sample liquid supply channel 15 and the transport liquid supply channel 17 join. The upstream side joining portion 19 is disposed at an upstream end of the main channel 11. Therefore, the sample liquid L1 passing through the sample liquid supply channel 15 and the transport liquid L2 passing through the transport liquid supply channel 17 join in the upstream side joining portion 19 and flows through the main channel 11.

As shown in Fig. 1, the control liquid channel 21 joins to a portion of the main channel 11 which is located on the downstream side of the auxiliary channels 13. In the present preferred embodiment, the control liquid channel 21 joins to the downstream end of the main channel 11. A control liquid L3 flows through the control liquid channel 21. The control liquid channel 21 is a channel for supplying the control liquid L3 to the common channel 25. The control liquid L3 is a liquid not containing fine particles, for example. The control liquid L3 is not particularly limited but is, for example, the same in components as the transport liquid L2. For example, the liquid component of the control liquid L3 is the same as the liquid component of the transport liquid L2. Also, for example, the conductivity of the control liquid L3 is the same as the conductivity of the transport liquid L2.

Here, by adjusting the amount of the control liquid L3 made to flow to the control liquid channel 21, it is possible to control the particle size of the particles flowing from the main channel 11 into the plurality of auxiliary channels 13. In other words, the particle size of the particles to be separated can be controlled by adjusting the amount of the control liquid L3 made to flow to the control liquid channel 21. It is noted that, in the present preferred embodiment, the control liquid channel 21 is perpendicular to the main channel 11, but the present invention is not limited thereto. For example, the control liquid channel 21 may be parallel to the main channel 11 or may be inclined with respect to the main channel 11. It is noted that a method for controlling the particle size of the particles to be separated by the control liquid channel 21 will be described later.

At the downstream side joining portion 23, the main channel 11 and the control liquid channel 21 join. The downstream side joining portion 23 is disposed at a downstream end of the main channel 11.

The common channel 25 is connected to the downstream side joining portion 23. Therefore, the liquid that has passed through the main channel 11 and the control liquid L3 that has passed through the control liquid channel 21 join at the downstream side joining portion 23 and flow through the common channel 25. At least particles larger than a predetermined particle size contained in the sample liquid L1 and the control liquid L3 flow through the common channel 25. In the present preferred embodiment, particles larger than a predetermined particle size, the control liquid L3, and the transport liquid L2 flow.

Successively with reference to FIG. 1, the separation device 100 and the HDF chip 1 will be further described. The separation device 100 further includes a sample liquid sending portion 31, a transport liquid sending portion 33, and a control liquid adjusting portion 35. The separation device 100 may include a control unit that drives the sample liquid sending portion 31, the transport liquid sending portion 33, and the control liquid adjusting portion 35. The sample liquid sending portion 31, the transport liquid sending portion 33, and the control liquid adjusting portion 35 are not particularly limited but are, for example, micropumps. The micropumps may be mechanical pumps or may be non-mechanical pumps.

The sample liquid sending portion 31 connects to the sample liquid supply channel 15 and causes the sample liquid L1 flow into the sample liquid supply channel 15. Specifically, the sample liquid supply channel 15 has a sample liquid inlet 15a. The sample liquid inlet 15a is disposed at an upstream end of the sample liquid supply channel 15. The sample liquid sending portion 31 connects to the sample liquid inlet 15a. By supplying the sample liquid L1 to the sample liquid inlet 15a by the sample liquid sending portion 31, the sample liquid L1 flows through the sample liquid supply channel 15 and the main channel 11.

The transport liquid sending portion 33 connects to the transport liquid supply channel 17 and causes the transport liquid L2 flow into the transport liquid supply channel 17. Specifically, the transport liquid supply channel 17 has a transport liquid inlet 17a. The transport liquid inlet 17a is disposed at the upstream end of the transport liquid supply channel 17. The transport liquid sending portion 33 is connected to the transport liquid inlet 17a. By supplying the transport liquid L2 to the transport liquid inlet 17a by the transport liquid sending portion 33, the transport liquid L2 flows through the transport liquid supply channel 17 and the main channel 11.

The control liquid adjusting portion 35 is connected to the control liquid channel 21 and causes the control liquid L3 to flow into the control liquid channel 21. Specifically, the control liquid channel 21 has a control liquid inlet 21a. The control liquid inlet 21a is disposed at the upstream end of the control liquid channel 21. The control liquid adjusting portion 35 is connected to the control liquid inlet 21a. By supplying the control liquid L3 to the control liquid inlet 21a by the control liquid adjusting portion 35, the control liquid L3 flows through the control liquid channel 21 and the common channel 25.

In the present preferred embodiment, the sample liquid sending portion 31 supplies the sample liquid L1 so that the sample liquid L1 becomes a laminar flow. The transport liquid sending portion 33 supplies the transport liquid L2 so that the transport liquid L2 becomes a laminar flow. The control liquid adjusting portion 35 supplies the control liquid L3 so that the control liquid L3 becomes a laminar flow. Therefore, in the present preferred embodiment, each liquid becomes a laminar flow in the HDF chip 1. It is noted that the control liquid adjusting portion 35 may supply the control liquid L3 so that the control liquid L3 does not become a laminar flow.

Also, in the present preferred embodiment, the control liquid adjusting portion 35 can adjust the amount of the control liquid L3 flowing through the control liquid channel 21. That is, the control liquid adjusting portion 35 can adjust the amount of the control liquid L3 to be supplied to the control liquid channel 21. It is noted that the sample liquid sending portion 31 may or may not be capable of adjusting the amount of the sample liquid L1 flowing through the sample liquid supply channel 15. Also, the transport liquid sending portion 33 may or may not be capable of adjusting the amount of the transport liquid L2 flowing through the transport liquid supply channel 17.

Also, in the present preferred embodiment, the control liquid adjusting portion 35 and the sample liquid sending portion 31 are driven independently of each other. Therefore, for example, the supply amount of the control liquid L3 can be changed regardless of the supply amount of the sample liquid L1. Also, the transport liquid sending portion 33 and the control liquid adjusting portion 35 are driven independently of each other. Therefore, for example, the supply amount of the control liquid L3 can be changed regardless of the supply amount of the transport liquid L2. It is noted that the sample liquid sending portion 31 and the transport liquid sending portion 33 may be driven independently of each other or may not be driven independently. Also, the sample liquid sending portion 31 and the transport liquid sending portion 33 may be the same pump.

The HDF chip 1 further includes a first outlet 27 and a second outlet 29. The first outlet 27 is connected to the plurality of auxiliary channels 13. The liquid having passed through the plurality of auxiliary channels 13 is recovered by a first recovery portion (not illustrated) via the first outlet 27. The first recovery portion includes a recovery container, for example. The second outlet 29 is connected to the downstream end of the common channel 25. The particles, the transport liquid L2, and the control liquid L3 that have passed through the common channel 25 are collected in a second recovery portion (not illustrated) via the second outlet 29. The second recovery portion includes a recovery container, for example.

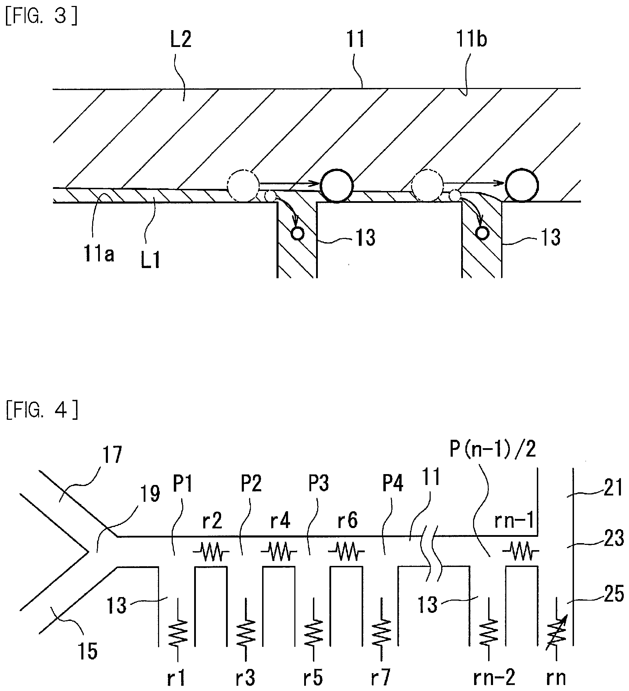

Next, with reference to FIG. 3, the operating principle of the HDF 2 will be described. FIG. 3 is a schematic view for explaining the operating principle of an HDF 2. As shown in FIG. 3, when the sample liquid sending portion 31 supplies the sample liquid L1 and the transport liquid sending portion 33 supplies the transport liquid L2, the sample liquid L1 and the transport liquid L2 flow through the main channel 11. The sample liquid L1 flows along the inner wall 11a on the auxiliary channels 13 side, and the transport liquid L2 flows along the inner wall 11b on the opposite side to the auxiliary channels 13. At this time, centers of various particles pass through positions separated from the inner wall 11a of the main channel 11 by not less than radiuses of the particles.

Here, the flow speed changes according to the position of the main channel 11 in the width direction. Specifically, the flow speed decreases near the inner wall 11a and the inner wall 11b of the main channel 11. On the other hand, the flow speed is the largest at a position far from the inner wall 11a and the inner wall 11b of the main channel 11 (the center of the main channel 11 in the width direction).

For this reason, since the flow speed of particles having a small particle size and passing near the inner wall 11a is low, the particles easily flow into the auxiliary channels 13. On the other hand, since the flow speed of particles having a large particle size and passing through a position far from the inner wall 11a is high, the particles do not flow into the auxiliary channels 13 and travel straight through the main channel 11. That is, under a predetermined condition such as the supply amount of the sample liquid L1 and the transport liquid L2 being constant, particles larger than the predetermined size are not discharged from the main channel 11, while particles of the predetermined size or less are discharged from the main channel 11. Since discharge from the main channel 11 to the auxiliary channels 13 is repeated by the plurality of auxiliary channels 13, the particles not more than the predetermined size do not reach the downstream end (the downstream side joining portion 23) of the main channel 11. It is noted that blood plasma serving as a liquid component does not reach the downstream end of the main channel 11 as well. Therefore, it is possible to separate the particles having the large particle sizes, such as cancer cells and the white blood cells, from blood by the HDF chip 1, for example.

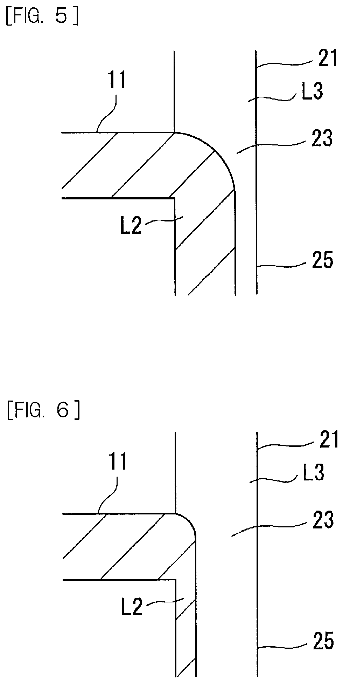

Next, a method of controlling the particle size of the particles to be separated using the control liquid channel 21 will be described with reference to FIGS. 4 to 6. FIG. 4 is a conceptual diagram for explaining channel resistance acting on the sample liquid L1 and the transport liquid L2 in the main channel 11, the auxiliary channels 13, and the common channel 25. FIGS. 5 and 6 are schematic views for explaining a relationship between the supply amount of the control liquid L3 and the amount of the transport liquid L2 flowing through the common channel 25.

As shown in Fig. 4, the channel resistances of the plurality of auxiliary channels 13 are set to r1, r3, r5, ..., rn-2 in order from the upstream side. The resultant channel resistance of the downstream side joining portion 23 and the common channel 25 is defined as rn. Here, n is a positive odd number. Also, the channel resistances of the main channel 11 between the auxiliary channels 13 are set to r2, r4, r6, ..., in order from the upstream side. Also, the channel resistance between the most downstream auxiliary channel 13 and the downstream side joining portion 23 is defined as rn-1.

In a branch portion P1 between the most upstream auxiliary channel 13 and the main channel 11, the ease of flowing to the auxiliary channel 13 is determined by the ratio between the channel resistance r1 and the combined resistance of the channel resistances r2 to rn. Similarly, In the branch portion P2 between the auxiliary channel 13 at the second to the upstream side and the main channel 11, the ease of flowing to the auxiliary channel 13 is determined by the ratio between the channel resistance r3 and the combined resistance of the channel resistances r4 to rn. The same applies thereafter.

Here, as shown in FIGS. 5 and 6, when the control liquid L3 flows into the control liquid channel 21, the amount of the transport liquid L2 flowing from the main channel 11 to the common channel 25 is limited. Specifically, when the amount of the control liquid L3 made to flow to the control liquid channel 21 is reduced, the channel area of the transport liquid L2 flowing through the common channel 25 increases (see FIG. 5). On the other hand, when the amount of the control liquid L3 made to flow to the control liquid channel 21 is increased, the channel area of the transport liquid L2 flowing through the common channel 25 decreases. As described above, the channel area of the transport liquid L2 made to flow to the common channel 25 is changed by changing the amount of the control liquid L3 flowing through the control liquid channel 21. In other words, the channel resistance rn acting on the transport liquid L2 is changed by changing the amount of the control liquid L3 made to flow to the control liquid channel 21.

Also, when the channel resistance rn changes, the combined resistance of the channel resistances r2 to rn changes. Therefore, since the ratio between the channel resistance r1 and the combined resistance of the channel resistances r2 to rn changes, the ease of flowing to the auxiliary channel 13 at the branch portion P1 changes. Similarly, since the ratio between the channel resistance r3 and the combined resistance of the channel resistances r4 to rn changes, the ease of flowing to the auxiliary channel 13 at the branch portion P2 changes. The same applies to the branch portions P3, P4, **...,** P(n-1)/2. It is noted that, for example, when the amount of the control liquid L3 made to flow to the control liquid channel 21 increases, the channel resistance rn increases. Therefore, for example, since the combined resistance of the channel resistances r2 to rn increases, the liquid easily flows from the branch portion P1 to the auxiliary channel 13. That is, for example, when the amount of the control liquid L3 made to flow to the control liquid channel 21 increases, the sample liquid L1 easily flows from the main channel 11 to the plurality of auxiliary channels 13.

As described above with reference to FIGS. 1 to 6, the separation device 100 includes the control liquid channel 21 which joins to the portion of the main channel 11 which is located on the downstream side of the auxiliary channel 13 and through which the control liquid L3 flows. Therefore, by changing the amount of the control liquid L3 made to flow to the control liquid channel 21, the ease of flowing to the auxiliary channel 13 at each of the branch portions P1, ..., changes. Therefore, for example, even in a case where the dimensions such as the width and depth of the channel at each position of the HDF chip 1 deviate from the set values due to manufacturing variations, by adjusting the amount of the control liquid L3 made to flow to the control liquid channel 21, it is possible to suppress particles (for example, cancer cells and white blood cells) larger than a predetermined particle size from flowing to the auxiliary channel 13 or particles (for example, red blood cells and platelets) having a predetermined particle size or less from passing through the main channel 11 and flowing to the common channel 25. Therefore, even if the dimension of the channel deviates from the set value, particles having a predetermined particle size can be separated.

Also, as described above, the separation device 100 includes the control liquid adjusting portion 35 capable of adjusting the amount of the control liquid L3 flowing through the control liquid channel 21. Therefore, the amount of the control liquid L3 made to flow to the control liquid channel 21 can be easily changed. Therefore, it is possible to easily suppress particles having a particle size larger than the predetermined particle size from flowing to the auxiliary channel 13 or particles having a predetermined particle size or less from flowing to the common channel 25.

Also, as described above, the control liquid adjusting portion 35 and the sample liquid sending portion 31 are driven independently of each other. Therefore, the supply amount of the control liquid L3 can be changed while the supply amount of the sample liquid L1 is maintained, for example, constant. Therefore, unlike the case where the supply amount of the sample liquid L1 is interlocked with the supply amount of the control liquid L3, the particle size of particles flowing to the auxiliary channel 13 and the particle size of particles flowing to the common channel 25 can be easily adjusted.

Also, as described above, the transport liquid sending portion 33 and the control liquid adjusting portion 35 are driven independently of each other. Therefore, the supply amount of the control liquid L3 can be changed while the supply amount of the transport liquid L2 is maintained, for example, constant. Therefore, unlike the case where the supply amount of the transport liquid L2 is interlocked with the supply amount of the control liquid L3, the particle size of particles flowing to the auxiliary channel 13 and the particle size of particles flowing to the common channel 25 can be easily adjusted.

Also, as described above, the control liquid L3 and the transport liquid L2 are the same in components. Therefore, it is possible to suppress a change in the liquid component of the liquid recovered at the second outlet 29 due to the supply amount of the control liquid L3.

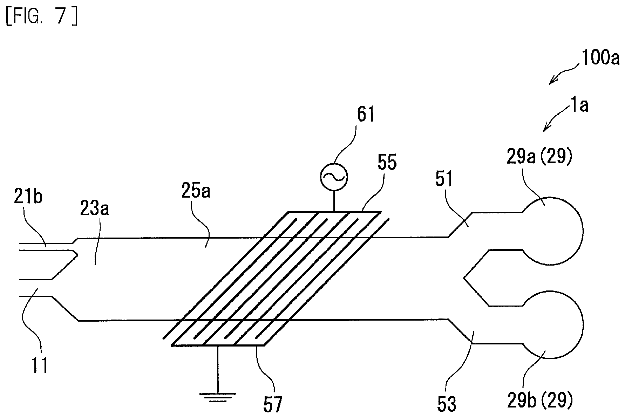

Next, a separation device 100a according to a modified example of the present invention will be described with reference to FIG. 7. FIG. 7 is a plan view schematically showing a structure of the separation device 100a according to a modified example of the present invention.

As illustrated in FIG. 7, in the separation device 100a according to the modified example of the present invention, an HDF chip 1a includes the main channel 11, a control liquid channel 21b, a downstream side joining portion 23a, a common channel 25a, a first recovery passage 51, and a second recovery passage 53. It is noted that the downstream side joining portion 23a is an example of a "first joining portion" of the present invention. Also, similar to the HDF chip 1, the HDF chip 1a includes the plurality of auxiliary channels 13, the sample liquid supply channel 15, the transport liquid supply channel 17, and the upstream side joining portion 19.

The main channel 11 and the control liquid channel 21b are arranged in parallel, for example. At the downstream side joining portion 23a, the main channel 11 and the control liquid channel 21b join. The width of the downstream side joining portion 23a is larger than the sum of the width of the main channel 11 and the width of the control liquid channel 21b. The width of the common channel 25a is larger than the sum of the width of the main channel 11 and the width of the control liquid channel 21b. The width of the common channel 25a is the same as the width of the downstream side joining portion 23a. Also, the depth of the main channel 11, the depth of the control liquid channel 21b, the depth of the downstream side joining portion 23a, and the depth of the common channel 25a are substantially equal. Therefore, the cross-sectional area of the downstream side joining portion 23a is larger than the sum of the cross-sectional area of the main channel 11 and the cross-sectional area of the control liquid channel 21b. Also, the cross-sectional area of the common channel 25a is larger than the sum of the cross-sectional area of the main channel 11 and the cross-sectional area of the control liquid channel 21b.

In this manner, by making the width of the common channel 25a larger than the sum of the width of the main channel 11 and the width of the control liquid channel 21b, the flow speed of liquid flowing through the common channel 25a can be reduced. Therefore, it is possible to easily separate the particles by the dielectrophoresis to be described later.

The first recovery passage 51 and the second recovery passage 53 are connected to the downstream end of the common channel 25a. That is, the common channel 25a branches into the first recovery passage 51 and the second recovery passage 53.

In the HDF chip 1a, the second outlet 29 includes a second outlet 29a and a second outlet 29b. The second outlet 29a connects to the downstream end of the first recovery passage 51, and the second outlet 29b connects to the downstream end of the second recovery passage 53.

Here, the HDF chip 1a can further separate the particles flowing through the common channel 25a by dielectrophoresis (DEP). Specifically, the HDF chip 1a further includes an electrode 55 and an electrode 57 arranged such as to overlap the common channel 25a. The electrode 55 and the electrode 57 are teeth-shaped electrodes opposing each other. The electrode 55 is connected to an AC source 61, for example. The electrode 57 is grounded, for example. In this modified example, the separation device 100a includes the AC source 61. It is noted that the separation device 100a does not have to include the AC source 61.

The teeth portions of the electrode 55 and the electrode 57 are inclined at, for example, 10° or more and 60° or less with respect to the common channel 25a. By applying AC voltage between the electrode 55 and the electrode 57 by the AC source 61, it is possible to cause the dielectrophoresis. By adjusting a frequency of the AC voltage to be applied, it is possible to separate and recover desired particles from plurality of types of particles passing through the common channel 25a.

Specifically, in a case where the plurality of types of particles passing through the common channel 25a contain dielectric particles, it is possible to separate particular dielectric particles from the plurality of types of particles by the DEP. For example, cancer cells and white blood cells are dielectric particles. In a case where the plurality of types of particles passing through the common channel 25a contain the cancer cells as well as particles larger than a predetermined size, the particles being other than the cancer cells (such as the white blood cells), AC voltage of a particular frequency is applied between the electrode 55 and the electrode 57 so that a dielectrophoresis force is made to easily act on the cancer cells, for example. Thereby, since it is possible to move the cancer cells along the electrode 55 and the electrode 57, it is possible to induce only the cancer cells to the second outlet 29a by the dielectrophoresis. Therefore, it is possible to separate the cancer cells and the other particles (such as the white blood cells).

Other structures of the separation device 100a, the HDF chip 1a, the control liquid channel 21b, the downstream side joining portion 23a, and the common channel 25a according to the modified example of the present invention are the same as those of the separation device 100, the HDF chip 1, the control liquid channel 21, the downstream side joining portion 23, and the common channel 25 according to the above preferred embodiment. Also, other effects of the modified example of the present invention are the same as the effects of the preferred embodiment described above.

The preferred embodiment of the present disclosure has been described above with reference to the drawings. However, the present disclosure is not limited to the preferred embodiment described above, and can be implemented in various modes within the range not departing from the gist thereof. Also, by appropriately combining the plurality of constituent elements disclosed in the preferred embodiment described above, it is possible to form various inventions. For example, some constituent elements may be deleted from all constituent elements shown in the preferred embodiment. Further, the constituent elements of different preferred embodiments may be appropriately combined. The drawings schematically show the respective constituent elements mainly in order to facilitate understanding, and the thickness, the length, the number, the interval, etc., of each constituent element shown in the figures may be different from the actual ones for convenience in creating the drawings. Also, the material, the shape, the size, etc. of each constituent element shown in the preferred embodiment described above are not particularly limited but just an example, and can be variously changed within the range substantially not departing from the effects of the present disclosure.

For example, in the preferred embodiment described above, the example in which blood is used as the sample liquid L1 and the liquid culture media is used as the transport liquid L2 is described, but the present invention is not limited thereto. For example, a liquid other than blood may be used as the sample liquid L1 and a liquid other than the liquid culture media may be used as the transport liquid L2.

Also, in the preferred embodiment described above, the particles to be recovered from the second outlet 29 (for example, the cancer cells and the white blood cells) are exemplified as a target to be recovered, but the present invention is not limited to this. For example, particles or a liquid component to be recovered from the first outlet 27 may be the target to be recovered.

Also, in the modified example described above, the example in which the target particles (for example, the cancer cells) are recovered from the second outlet 29a is described, but the present invention is not limited to this. For example, the target particles may be recovered from the second outlet 29b.

Also, in the above preferred embodiment, an example in which the transport liquid L2 and the control liquid L3 do not contain particles is described, but the present invention is not limited thereto. The transport liquid L2 and the control liquid L3 may contain particles. However, in this case, it is preferable to provide the structure as described with reference to Fig. 7 and separate the particles contained in the transport liquid L2 and the control liquid L3 from the particles contained in the sample liquid L1.

Also, in the above preferred embodiment, the example of using the transport liquid L2 has been described. That is, the example in which the transport liquid L2 is made to flow to the main channel 11 in addition to the sample liquid L1 is described. However, the present invention is not limited thereto. For example, only the sample liquid L1 may flow into the main channel 11. For example, seawater containing particles may be used as the sample liquid L1 to separate the particles from the seawater.

Also, in the above preferred embodiment, the example in which the control liquid adjusting portion 35 can adjust the amount of the control liquid L3 flowing through the control liquid channel 21 is described, but the present invention is not limited thereto. For example, a plurality of types of control liquid adjusting portions 35 may be prepared, and particles may be separated from the sample liquid L1 using one of the control liquid adjusting portions 35 which supplies an appropriate amount of the control liquid L3.

### Industrial Applicability

The present invention is applicable to the field of separation devices.

### Reference Signs List

11 : main channel
13 : auxiliary channel
15 : sample liquid supply channel
17 : transport liquid supply channel
19 : upstream side joining portion (second joining portion)
21, 21b : control liquid channel
23, 23a : upstream side joining portion (first joining portion)
25, 25a : common channel
31 : sample liquid sending portion
33 : transport liquid sending portion
35 : control liquid adjusting portion
100, 100a : separation device
L1 : sample liquid
L2 : transport liquid
L3 : control liquid

## Claims

1. A separation device comprising:
a main channel through which a sample liquid containing particles flows;
a plurality of auxiliary channels branching from the main channel;
a control liquid channel joined to a portion of the main channel which is located on a downstream side of the auxiliary channels and through which a control liquid flows;
a first joining portion at which the main channel and the control liquid channel join; and
a common channel connected to the first joining portion,
wherein at least the particles larger than a predetermined particle size and the control liquid flow through the common channel.

2. The separation device according to claim 1, further comprising a control liquid adjusting portion capable of adjusting an amount of the control liquid flowing through the control liquid channel.

3. The separation device according to claim 2, further comprising a sample liquid sending portion that causes the sample liquid to flow into the main channel,
wherein the control liquid adjusting portion and the sample liquid sending portion are driven independently of each other.

4. The separation device according to claim 2 or 3, further comprising:
a sample liquid supply channel through which the sample liquid flows;
a transport liquid supply channel through which a transport liquid that transports the particles to the common channel flows;
a second joining portion at which the sample liquid supply channel and the transport liquid supply channel join; and
a transport liquid sending portion that causes the transport liquid to flow into the transport liquid supply channel,
wherein the transport liquid sending portion and the control liquid adjusting portion are driven independently of each other.

5. The separation device according to claim 4, wherein the control liquid and the transport liquid are the same in components.

6. The separation device according to any one of claims 1 to 5, wherein a width of the common channel is larger than a sum of a width of the main channel and a width of the control liquid channel.
